# EUROPÄISCHE PATENTSCHRIFT

(11) **EP 0 887 643 B1**
(45) Veröffentlichungstag und Bekanntmachung des Hinweises auf die Patenterteilung: **22.05.2002**
(21) Anmeldenummer: 98250232.0
(22) Anmeldetag: 24.06.1998
(51) Int. Cl.: G01N 33/18, G01N 31/12

(54) **Verfahren zur Bestimmung eines Wasserinhaltsstoffes**
Method of determining components in aqueous systems
Procédé de détermination de la teneur de composants dans des systèmes aqueux

(30) Priorität: 24.06.1997 DE 19727839
(43) Veröffentlichungstag der Anmeldung: 30.12.1998
(73) Patentinhaber: LAR Analytik und Umweltmesstechnik GmbH, 10179 Berlin (DE)
(72) Erfinder: Pilz, Ulrich, Dr.-Ing., 13465 Berlin (DE)
(74) Vertreter: Heinze, Ekkehard, Dipl.-Phys. Dr.

(56) Entgegenhaltungen:
- EP-A- 0 660 110
- EP-A- 0 684 471
- DE-A- 2 934 561
- DE-A- 4 309 646
- DE-C- 4 344 441
- US-A- 3 840 341
- US-A- 4 977 094
- US-A- 5 292 666

## Beschreibung

Die Erfindung betrifft ein Verfahren zur Bestimmung von Wasserinhaltsstoffen gemäß dem Oberbegriff des Anspruchs 1 sowie eine Vorrichtung zur Durchführung des Verfahrens.

Es ist bekannt, zur Bestimmung des Gehaltes an bestimmten Wasserinhaltsstoffen - und damit der Qualität von Wasser und insbesondere von durch organische Stoffe und/oder Stickstoffverbindungen und/oder Halogenverbindungen belastetem Abwasser - eine Probe in einer Atmosphäre eines mit Sauerstoff angereicherten inerten Transportgases zu verdampfen und zu verbrennen und das hierbei erhaltene Verbrennungsgasgemisch einem zum Nachweis von Kohlendioxid, Stickoxiden etc. geeigneten Detektor zuzuführen.

Als Detektoren haben sich (neben anderen) Infrarotdetektoren für den Kohlenstoffgehalt, spezielle Chemolumineszenzdetektoren für den Stickoxidgehalt und sogenannte coulometrische Detektoren für den Halogenidgehalt bewährt.

Große Verbreitung haben die auf der Verbrennung einer Wasserprobe beruhenden Nachweisverfahren für die Erfassung des Gehaltes an organischen Inhaltsstoffen - des sogenannten TOC (total organic carbon) - erlangt. Hierbei wird üblicherweise eine kleine Wassermenge mit dem Transportgas einem mit einer Widerstandsheizung auf eine vorbestimmte Temperatur aufgeheizten Ofen zugeführt, wo sie nahezu schlagartig verdampft und verbrennt, und das Verbrennungsgas wird einem NDIR-CO₂-Detektor zugeführt, dessen CO₂-Gehaltsanzeige ein Maß für den C-Gehalt der Wasserprobe bildet. Eine fortgeschrittene Ausführung dieses Verfahrens und eine entsprechende Apparatur sind in DE 43 44 441 C2 beschrieben. Eine zur Messung sehr niedriger TOC-Werte - etwa in hochreinem Wasser bzw. hochreinen Lösungen für medizinische Anwendungen - modifizierte Anordnung ist in EP 0 684 471 A2 beschrieben.

Mit diesen Verfahren wird nicht ohne weiteres der interessierende TOC, sondern grundsätzlich der Gesamt-Kohlenstoffgehalt des Wassers (TC = total carbon) bestimmt, der neben dem TOC den Anteil an anorganischen Kohlenstoffverbindungen (TIC = total inorganic carbon) umfaßt. Diese werden daher zur Bestimmung des TOC in einem vorgeschalteten Abtrennschritt (sog. Strippen) abgetrennt; vgl. dazu etwa DE 39 42 229 C2 (mit weiteren Literaturhinweisen).

Bei der Abtrennung des anorganischen Kohlenstoffs durch Austreiben tritt das weitere Problem auf, daß austreibbarer bzw. flüchtiger organischer Kohlenstoff (POC bzw. VOC = volatile organic carbon) ebenfalls aus der Probe entfernt wird. In DE 43 09 646 A1 werden daher ein Verfahren und eine Untersuchungsanordnung des oben skizzierten Typs vorgeschlagen, bei denen der POC-Gehalt getrennt gemessen und zur Gewinnung korrekter POC-Meßwerte ungewollt mit ausgetriebenen Kohlenstoffverbindungen durch ein spezielles Adsorberagens abgefangen wird.

In der praktischen Durchführung kann andererseits für feststoffhaltige Proben nicht der gesamte Gehalt an organischen Inhaltsstoffen bestimmt werden, was die Meßergebnisse zusätzlich verfälscht. Zur Vermeidung zu starker stoßartiger Druckbelastungen der Analysenapparatur durch ein Verpuffen im heißen Ofen müssen nämlich die zugeführten Wasserprobenmengen sehr klein gemacht werden, was den Einsatz feinster Dosiertechnik voraussetzt. Zu deren Schutz wiederum muß eine Feinfiltration der Probe vorgenommen werden, mit der ein wesentlicher Teil der Feststoffe und damit der Gesamtmenge an organischen Inhaltsstoffen von der Gehaltsbestimmung ausgeschlossen wird. Die infolge der geringen Probemengen ohnehin schon mäßige Nachweisgenauigkeit wird dadurch in für viele Anwendungen, beispielsweise die Abwaserranalyse, inakzeptabler Weise verschlechtert.

In gewissem Umfang schafft beim letztgenannten Problem ein Verfahren Abhilfe, bei dem in diskontinuierlichen Analysevorgängen jeweils eine wäßrige Probe in einem anfänglich kalten Heizgefäß relativ langsam erwärmt und verbrannt wird; vgl. DE 44 12 778 C1. Der CO₂-Gehalt des Verbrennungsgases muß bei diesem diskontinierlichen Verfahren über eine bestimmte Zeitspanne verfolgt und integriert werden. Dieses Verfahren vermeidet starke Druckschwankungen in der Apparatur, und daher können größere Probenmengen zum Einsatz kommen. Jedoch erfordert hierbei die notwendige Verfolgung der Zeitabhängigkeit der CO₂-Konzentration bis in den Bereich sehr niedriger Werte einen hochempfindlichen und entsprechend teuren Detektor, der auch in diesem Bereich noch genau arbeitet sowie einen Präzisionsintegrator. Der apparative Aufwand erhöht sich weiter durch das Vorsehen einer zweiten Heizeinrichtung.

Der Erfindung liegt deshalb die Aufgabe zugrunde, ein kostengünstiges und für den Routinebetrieb geeignetes Verfahren mit hoher Meßgenauigkeit zur spezifischen Bestimmung von Wasserinhaltsstoffen, insbesondere des TOC, sowie eine Vorrichtung zur Durchführung des Verfahrens anzugeben.

Die Aufgabe wird hinsichtlich des Verfahrensaspekts durch ein Verfahren mit den Merkmalen des Anspruchs 1 und hinsichtlich des Vorrichtungsaspekts durch eine Vorrichtung mit den Merkmalen des Anspruchs 15 gelöst.

Die Erfindung schließt den Gedanken ein, das bekannte quasistationäre Verfahren, das auf der Verdampfung und Verbrennung sehr kleiner Wassermengen in einem permanent auf Verbrennungstemperatur gehaltenen Ofen beruht, durch ein dynamisches Zwei-Stufen-Verfahren mit einzelnen Verdampfungs- und Verbrennungsvorgängen zu ersetzen. Dank einer speziellen Verfahrensführung ist dennoch nicht die Erfassung sehr niedriger CO₂-Konzentrationen und somit auch nicht der Einsatz eines hochempfindlichen, teuren Detektors erforderlich.

In einer vorteilhaften Variante der Erfindung ist vorgesehen, daß die Verdampfungstemperatur im Bereich zwischen 100°C und 300°C, bevorzugt zwischen 100°C und 150°C, und die Verbrennungstemperatur oberhalb 700°C, bevorzugt zwischen 800°C und 1000°C, liegt.

Die Erwärmung auf die Verdampfungstemperatur im ersten Schritt kann etwa in einer Zeitspanne zwischen 10 s und 200 s und die Erwärmung auf die Verbrennungstemperatur im zweiten Schritt in einer Zeitspanne zwischen 5 s und 120 s vorgenommen werden. Lange Zeitspannen ermöglichen die Umsetzung relativ großer Probenvolumina in die Meßapparatur schonender Weise und sind dann vorzuziehen, wenn keine besonders kurze Zykluszeit für die Messungen erforderlich ist. Im Anschluß an den ersten Schritt wird die Temperatur vorteilhafterweise für eine vorbestimmte Zeitspanne, bevorzugt zwischen 10s und 60s, im wesentlichen konstant gehalten.

Speziell für die TOC-Messung wird das im zweiten Schritt erhaltene Verbrennungsprodukt einem CO₂-Detektor, insbesondere einem NDIR-Detektor, zur Bestimmung des Kohlenstoffgehaltes zugeführt. Den Schritten des Verdampfens und Verbrennens geht hierbei ein Schritt der Separierung anorganischer Kohlenstoffverbindungen aus der Probe, insbesondere durch saure Entgasung, voran, da diese den CO₂-Meßwert unter Vortäuschung eines zu hohen TOC verfälschen würden.

In Verbindung mit dem Schritt der Separierung der anorganischen Kohlenstoffverbindungen kann in vorteilhafter Weise sogleich eine Bestimmung des Gehalts an anorganischem Kohlenstoff, insbesondere durch Zuführung des Entgasungsprodukts zu dem CO₂-Detektor vor dem Schritt des Verbrennens, durchgeführt werden. Ist diese Quantifizierung aber verzichtbar, so können diese Kohlenstoffverbindungen auch ohne weiteres durch einen CO₂-Fänger ausgeschieden werden.

Besteht das Anliegen einer Bestimmung des Gehaltes an Stickstoffverbindungen, so wird das im zweiten Schritt erhaltene Verbrennungsprodukt einem Stickstoffdetektor, insbesondere einem Chemolumineszenzdetektor, zur Bestimmung des Stickoxidgehaltes im Verbrennungsgas-/Trägergas-Gemisch zugeführt.

Zum Schutz der Meßapparatur vor Korrosion ist für die meisten praktischen Anwendungen dem Schritt des Verdampfens ein Schritt der Abtrennung von Halogenid-, insbesondere Chlorid-Ionen nachgeschaltet. In Verbindung mit dem Schritt der Abtrennung kann bedarfsweise zugleich eine Gehaltsbestimmung der Halogenidionen - insbesondere mittels eines elektrolytischen (coulometrischen) Verfahrens sogleich am Abtrennungs-Reagens - durchgeführt werden.

Das im Schritt des Verdampfens erhaltene Gas, das im wesentlichen aus Wasserdampf besteht, wird in einer Kühlfalle zu einer wäßrigen Lösung kondensiert, und der Schritt der Separierung von Halogenidionen wird mittels eines geeigneten Agens, insbesondere AgNO₃-Lösung oder Silberwatte, in der Kühlfalle ausgeführt.

Eine Vorrichtung zur Durchführung des oben skizzierten Verfahrens weist als Heizeinrichtung einen trägheitsarm zyklisch zu betreibenden Ofen mit geringerthermischer Masse, insbesondere einen optischen (Infrarot-)Strahlungsofen, auf, der mit einer schnell ansprechenden Temperatur-Zeit-Steuerung versehen ist.

Ein geeigneter Strahlungsofen weist eine Mehrzahl von auf einer Zylindermantelfläche um das Reaktionsgefäß herum angeordneten Halogenheizstäben und einen die Halogenheizstäbe umgebenden zylindrischen Reflektor auf. In einer alternativen Ausführung umfaßt der Strahlungsofen einen elliptischen Reflektor, in dessen einer Brennlinie das Reaktionsgefäß und in dessen anderer Brennlinie ein Halogenheizstab angeordnet ist.

Die Meßapparatur insgesamt ist in bevorzugter Weise als gegenüber der Atmosphäre abschließbares verzweigtes System ausgebildet, das mindestens eine das Reaktions- Bzw. Verbrennungsgefäß mit dem Detektor verbindende Ringleitung umfaßt. Bei Ausbildung zur TOC-Bestimmung umfaßt die Apparatur weiter eine ausgangsseitig mit dem Detektor verbundene Entgasungseinrichtung zur sauren Entgasung der Probe und einen in eine Leitung zwischen der Entgasungseinrichtung und dem Detektor einschleifbaren CO₂-Fänger.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: eine schematische Darstellung einer Anordnung zur Durchführung eines Verfahrens gemäß einer ersten Ausführungsform der Erfindung,
- Figur 2: eine schematische Darstellung einer gegenüber Fig. 1 modifizierten Anordnung zur Realisierung einer zweiten Ausführungsform,
- Figur 3: eine schematische Darstellung einer weiteren modifizierten Anordnung zur Realisierung einer dritten Ausführungsform und
- Figuren 4a und 4b: Prinzipskizzen zweier Ausführungsformen eines im Rahmen der Ausführung der Erfindung vorteilhaft einsetzbaren Strahlungsofens.

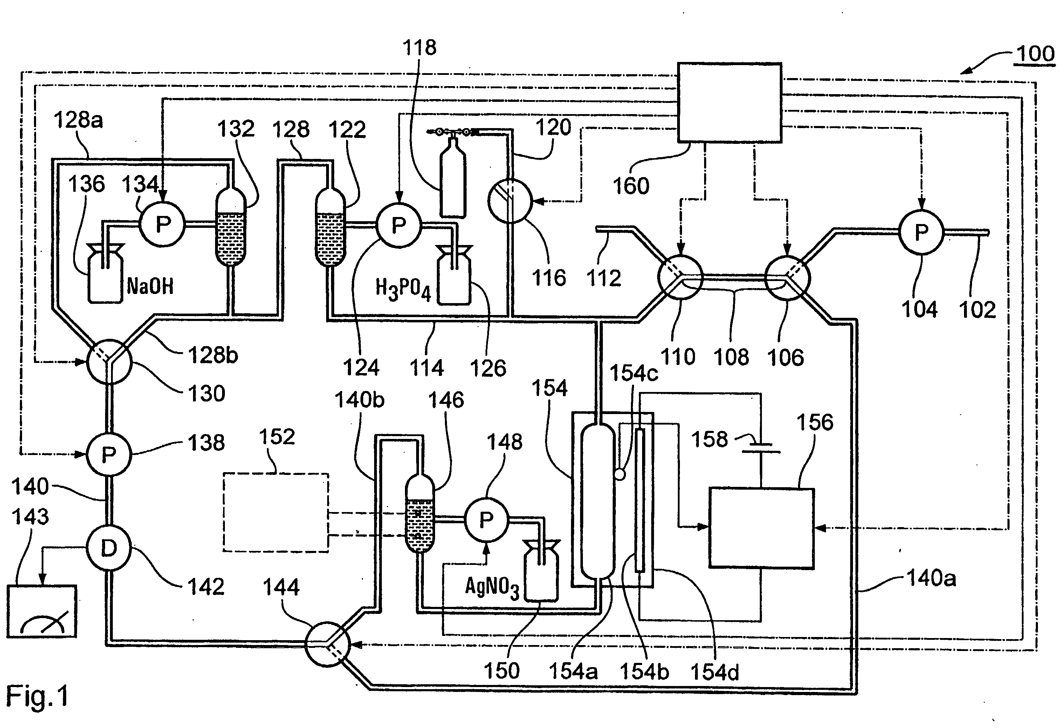

Fig. 1 zeigt eine TOC-Meßanordnung 100 zur Bestimmung des gesamten organischen Kohlenstoffgehalts einer (in der Figur nicht dargestellten) wäßrigen Probe. Die Anordnung umfaßt einen Probenansaugstutzen 102 zur Probenahme über eine Ansaugpumpe 104. Der Probenansaugstutzen 102 mündet stromabwärts der Pumpe 104 in ein Zweiwegeventil 106, das seinerseits in einen Leitungsabschnitt 108 mit exakt definierten Abmessungen (Querschnitt und Länge) und mündet, der stromabwärts durch ein weiteres Zweiwegeventil 110 begrenzt ist. Ein Ansaugen von Probenlösung mittels der Ansaugpumpe 104 in den Leitungsabschnitt 108 bei zum Ansaugstutzen 102 hin geöffnetem Ventil 106 und geschlossenem Ventil 110 fördert ein durch die Abmessungen des Leitungsabschnitts 108 vorbestimmtes Probenvolumen von beispielsweise 2-5 ml in die Vorrichtung 100.

Vom Ventil 110 gehen ein Ablaßstutzen 112 und eine Verbindungsleitung 114 ab, in die über ein Absperrventil 116 eine mit einem Sauerstoffbehälter 118 verbundener Gasleitung 120 mündet und die ihrerseits in eine erste Gaswaschflasche 122 mündet. Die Waschflasche 120 mit einem Fassungsvermögen von ca. 10 ml dient zur sauren Entgasung der Probe mittels einer darin vorgelegten H₃PO₄-Lösung, die über eine Dosierpumpe 124 aus einem Vorratsgefäß 126 zugeführt wird; siehe dazu auch weiter unten. Von der Waschflasche 122 geht eine weitere Verbindungsleitung 128 ab, die sich in zwei Leitungszweige 128a und 128b verzweigt, welche zu zwei Anschlüssen eines weiteren Zweiwegeventils 130 führen. Im Zweig 128a ist eine weitere Gaswaschflasche 132 mit ca. 10 ml Nutzvolumen angeordnet, in die über eine Dosierpumpe 134 aus einem Vorratsbehälter 136 NaOH-Lösung dosiert werden kann und die - in weiter unten genauer dargestellter Weise - als CO₂-Fänger dient. In der Praxis muß die während einer Messung verbrauchte NaOH-Lösung danach verworfen werden, was durch Absaugen in ein Sammelgefäß erfolgen kann; die hierzu erforderlichen Komponenten sind der besseren Übersichtlichkeit halber in der Figur aber nicht dargestellt.

Der Ausgang des Zweiwegeventils 130 ist über eine Förderpumpe 138 und eine weitere Verbindungsleitung 140, in die ein NDIR-CO₂-Detektor 142 eingeschleift ist, mit einem weiteren Zweiwegeventil 144 verbunden, das in zwei Zweig-Leitungen 140a und 140b mündet. Während die Leitung 140a direkt zum zweiten Eingang des oben erwähnten Zweiwegeventils 106 führt, ist in der in die Leitung 114 mündenden Leitung 140b nahe dem Ventil 144 zunächst eine weitere Waschflasche 146 vorgesehen, die über eine Dosierpumpe 148 mit einem Vorratsbehälter 150 für AgNO₃-Lösung verbunden ist. Diese dient, wie weiter unten näher erläutert wird, als Wasserdampfkondensator und Chloridfänger und wird ebenfalls beim Meßprozeß verbraucht; die ihrem Verwerfen dienenden Komponenten sind jedoch wiederum nicht dargestellt. Dem Chloridfänger ist optional - hier lediglich als gestrichelt gezeichneter Block dargestellt - eine an sich bekannte coulombmetrische TOX-Meßeinrichtung 152 zur Erfassung des Halogenidgehaltes zugeordnet.

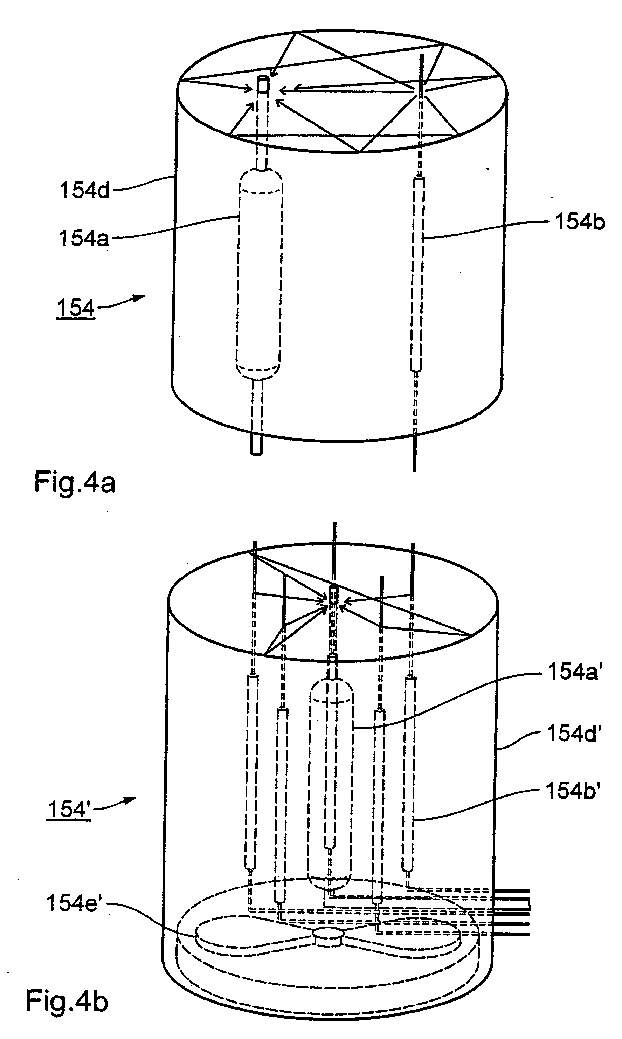

Zwischen dem Kondensator/Chloridfänger 146 und der Einmündung in die Leitung 114 ist ein Strahlungsofen 154 zur Verdampfung und Verbrennung der Probe angeordnet. Dieser umfaßt ein Reaktionsgefäß 154a und einen über eine Heizungs-Ansteuerung 156 mit einer Heizstromquelle 158 verbundenen Heizstab 154b sowie einen Temperaturfühler 154c in einem innenreflektierenden Gehäuse 154d; vgl. auch Fig. 4a. Sein Ausgang mündet in die oben erwähnte Verbindungsleitung 114.

Die gesamte Vorrichtung wird durch eine Mikroprozessor-Programmsteuerung (Controller) 160 gesteuert. Der Ablauf des Meßverfahrens ist grundsätzlich wie folgt:

Nach einer vorangehenden Messung erfolgt zunächst ein Verwerfen des verbrauchten Inhalts der Waschflaschen 132 und 146 und ein Befüllen aller Waschflaschen 122, 132 und 146. Weiterhin wird die Anordnung - unter Absperrung des Leitungszweiges 140b mit dem Chloridfänger 146 und dem Ofen 154 über das Ventil 144 - bei geöffnetem Absperrventil 116 und zum Ablaufstutzen 112 geöffnetem Ventil 110 mit Sauerstoff gespült. Durch Öffnen und anschließendes Schließen der Probenventile 106 und 110 und Einschalten der Pumpe 104 wird der Leitungsabschnitt 108 mit frischer Probenlösung gespült und ein definiertes Probenvolumen für die Messung bereitgestellt.

Anschließend werden das Probenansaugventil 106 und das O₂-Ventil 116 geschlossen und der die Probe enthaltende Leitungsabschnitt 108 durch Öffnen des Ventils 110 zur Leitung 114 hin in die Anordnung eingeschleift, wobei das Ventil 130 zum Leitungszweig 128b hin und das Ventil 144 zum Leitungszweig 140a hin offen ist und die Leitungszweige 128a und 140b somit abgesperrt bleiben. Durch Einschalten der Förderpumpe 138 wird die Probe in die Waschflasche 122 zur sauren Entgasung gefördert.

Beim Durchströmen der H₃PO₄-Lösung werden die in der Probe enthaltenen anorganischen Kohlenstoffverbindungen (gelöstes CO₂, H₂CO₃, Hydrogencarbonate und Carbonate), die den TIC ergeben, in im Probenwasser gelöstes CO₂ überführt. Die Durchmischung mit dem Sauerstoff im Leitungssystem bewirkt das sogenannte Strippen, d.h. das CO₂ wird in die Gasphase übernommen und kann in dieser durch den Infrarotdetektor 142 nachgewiesen werden. Auf diese Weise kann zunächst der TIC-Gehalt der Probe bestimmt werden. Wenn das Volumen des Gaskreislaufs exakt bekannt ist, kann dabei eine Quantifizierung des TIC im Sinne einer Absolutmessung erfolgen.

Anschließend wird durch Umlegen des Ventils 130 auf den Leitungszweig 128a der CO₂-Fänger 132 in den Kreislauf eingeschaltet und zugleich der "Bypass" 128b abgesperrt und hierdurch das CO₂ aus dem Kreislaufgas entfernt. Ist die separate Erfassung des TIC verzichtbar, kann von vornherein mit dieser Ventilstellung gearbeitet werden, wodurch Analysenzeit eingespart werden kann.

Ist das Kreislaufgas über die saure Entgasung und den CO₂-Fänger vollständig vom anorganischen Kohlenstoff gereinigt (was am CO₂-Detektor 142 überwacht oder aus dem Ablauf einer erfahrungsgemäß benötigten Zeitspanne geschlossen werden kann), wird der CO₂-Fänger durch erneutes Umschalten des Ventils 130 wieder ausgeschaltet und durch Umschalten des Ventils 144 anstelle des Leitungszweiges 140a der Zweig 140b mit dem Ofen 154 und dem Chloridfänger 146 in den Kreislauf geschaltet. Durch Einschalten der Förderpumpe 138 mit umgekehrter Förderrichtug wird die TIC-freie Probe einschließlich der vorgelegten Phosphorsäure in den Ofen 154 gedrückt. Dann wird zunächst eine stabile Einstellung des CO₂-Basiswertes am CO₂-Detektor 142 abgewartet. (Dies kann sowohl durch Bedienpersonal als auch mittels eines automatischen Meßwertvergleichs über den Controller 160 geschehen. Im letzteren Falle muß natürlich eine Datenverbindung vom Detektor 142 zum Controller 160 bestehen.)

Anschließend wird der Ofen 154 unter Programmsteuerung durch den Controller 160 und die Heizsteuerung 156 zunächst auf eine erste Temperatur zum relativ langsamen Verdampfen des Wassers (z.B. 120°C) aufgeheizt. Der Wasserdampf wird im Kondensator/Chloridfänger 146 kondensiert und zugleich zum Schutz der Anordnung gegen Korrosion durch Ausfällen von AgCl von Cl⁻-Ionen befreit. In gleicher Weise können auch J⁻ und Br⁻-Ionen gebunden werden. Nach Abschluß des Verdampfens nach 10-200 Sekunden wird der Ofen in einer zweiten Aufheizphase in wenigen Sekunden auf 800 - 900°C aufgeheizt und hierdurch binnen ca. 30 s der nach dem Verdampfen des Wassers verbliebene Teil der Probe verbrannt. Auch hierbei werden die Cl⁻-und ggfs. sonstigen Halogenidionen im Chloridfänger 146 gebunden.

Im Zuge der Verdampfung und anschließenden Verbrennung werden die gesamten organischen Kohlenstoffanteile der Probe (TOC unter Einschluß der flüchtigen Anteile - VOC) in CO₂ umgewandelt und im NDIR-Detektor 142 integrierend erfaßt. Da das Gaskreislauf- und das Probenvolumen bekannt ist, ist eine Absolutmessung mit hoher Genauigkeit möglich. Durch die integrierende Messung im geschlossenen Probe/Gas-Kreislauf wird die beim Stand der Technik bestehende Notwendigkeit der genauen Erfassung sehr kleiner CO₂-Konzentrationen im Abklingbereich der Verbrennung und damit der Einsatz eines teuren hochempfindlichen Detektors vermieden.

Der Strahlungsofen 154 ist aufgrund seiner geringen thermischen Masse schnell abgekühlt und für einen neuen Meßvorgang bereit, was kurze Meßzykluszeiten ermöglicht. Er kann zusätzlich ein Gebläse zur noch schnelleren Abkühlung aufweisen, wie in Fig. 4b gezeigt. Grundsätzlich ist in der Anordnung 100 jedoch auch ein anderer Ofen mit geringer thermischer Trägheit einsetzbar.

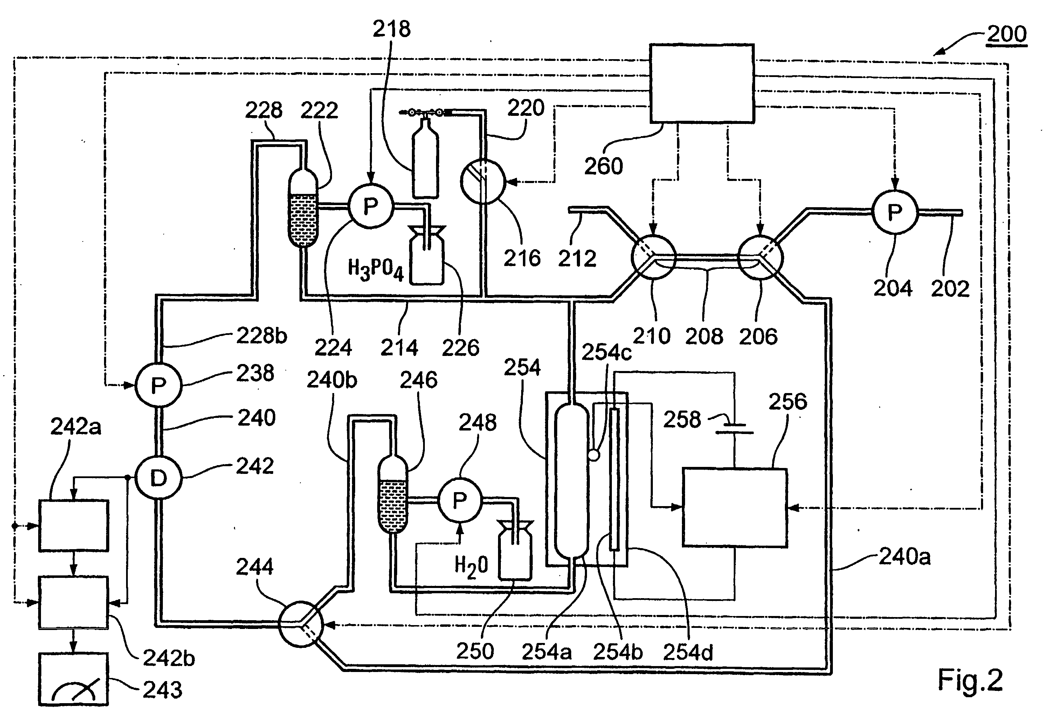

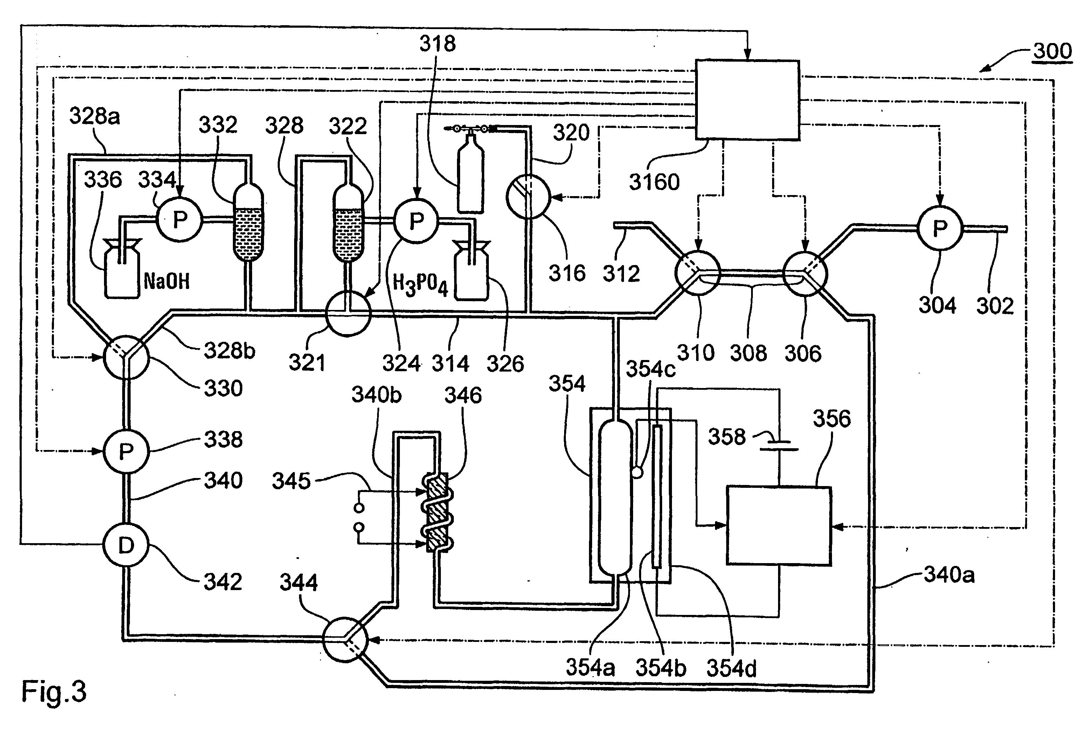

In Fig. 2 und 3 sind Abwandlungen der oben beschriebene Anordnung gezeigt, wobei gleiche oder ähnliche Teile wie in Fig. 1 mit korrespondierenden Ziffern bezeichnet sind und nachfolgend nicht nochmals erläutert werden.

Die Meßanordnung 200 nach Fig. 2 ist in ihrem Aufbau zunächst dadurch vereinfacht. daß auf den CO₂-Fänger mit Zubehörteilen sowie den entsprechenden Leitungszweig und das zugehörige Zweiwegeventil verzichtet wird. In die CO₂-Meßwerte geht hier notwendigerweise der TIC als Basissignal mit ein. Da dieser aber nach obigem Vorgehen vor dem Schritt des Verdampfens und Verbrennens der Probe separat bestimmt werden kann, ist bei dieser Anordnung die Bestimmung des TOC auf subtraktivem Wege möglich, wozu dem Detektor zusätzlich ein Meßwertspeicher 142a und eine Subtraktionsstufe 142b zugeordnet sind, die über den Controller 160 gesteuert werden. Weiterhin wirkt hier die Waschflasche 146 ausschließlich als Kühlfalle und wird statt mit AgNO₃ in betriebskostensparender Weise mit (hochreinem) Wasser befüllt. Dementsprechend ist naturgemäß auch keine Einrichtung zur Bestimmung des Halogenidgehaltes (TOX) vorgesehen.

Bei der Meßanordnung 300 nach Fig. 3 ist die als Kondensator/Chloridfänger wirkende Waschflasche 146 gemäß Fig. 1 durch einen Kühlkörper 346 mit Peltierkühler 345 ersetzt, und die CO2-Meßwerte werden vom IR-Spektrometer 342 zur Verarbeitung und Ausgabe einer Steuer- und Verarbeitungseinheit 360 übergeben. In einer weiteren Abwandlung, die auch eine Cl⁻-Abtrennung ermöglicht, kann der gekühlte Rohrabschnitt mit Ag-Watte gefüllt sein. Ein zusätzliches Magnetventil 321 zwischen den Leitungsabschnitten 314 und 328b ermöglicht bei der Anordnung 300 eine zeitweilige Umgehung der sauren Entgasung insgesamt, womit eine vom TOC getrennte Erfassung der flüchtigen Kohlenstoffanteile (VOC) in einem zusätzlichen Meßschritt möglich wird.

Die Erfindung beschränkt sich in ihrer Ausführung nicht auf die vorstehend angegebenen bevorzugten Ausführungsbeispiele. Vielmehr ist eine Anzahl von Varianten denkbar, welche von der dargestellten Lösung auch bei anders gearteten Ausführungen Gebrauch machen.

So muß nicht unbedingt ein einziges Verdampfungs- und Verbrennungsgefäß eingesetzt werden, sondern die Erhitzung der Probe kann stufenweise auch in zwei getrennten Gefäßen bzw. "Öfen" erfolgen, von denen das erste eine trägheitsarm steuerbare Heizeinrichtung im Sinne der obigen Beschreibung aufweist, während das zweite - der eigentlichen Verbrennung dienende - bevorzugt im wesentlichen auf konstanter Temperatur gehalten wird. Die Temperatur des ersten Gefäßes wird dann zwischen einer Verdampfungstemperatur und einer deutlich über dieser liegenden Temparatur (vorzugsweise oberhalb von ca. 400°C) verändert.

Im übrigen kann insbesondere das eigentliche Verbrennungsgefäß, also das einzige oder aber das zweite Erhitzungsgefäß, einen an einen Träger gebundenen geeigneten Katalysator (z.B. Be) aufweisen.

Weiterhin können wesentliche Teile der beschriebenen Anordnungen - mit einem etwa auf Chemolumineszenz-Basis arbeitenden Stickoxid-Detektor anstelle des CO₂-Detektors oder zusätzlich zu diesem - in ähnlicher Weise zur Bestimmung des Gesamt-Stickstoffgehaltes einer wäßrigen Probe (TN) genutzt werden.

Als CO₂-Detektor muß nicht notwendigerweise ein NDIR-Spektrometer eingesetzt werden; alternativ kann beispielsweise eine Überführung des CO₂ in Kohlensäure und der Einsatz eines pH-Sensors erfolgen.

## Patentansprüche

1. Verfahren zur Bestimmung eines Wasserinhaltsstoffes, insbesondere des Gehaltes an organischem Kohlenstoff und/oder Stickstoff, bei dem eine wäßrige Probe in mindestens einem mit einer Heizeinrichtung (154; 154'; 254; 354) versehenen Erhitzungsgefäß (154a; 154a'; 254; 354) verdampft und verbrannt und das Verbrennungsprodukt in einem Transportgasstrom einem Detektor (142; 242; 342) zur Konzentrationsbestimmung einer gasförmigen Verbindung des Inhaltsstoffes zugeführt wird, wobei das oder ein erstes Erhitzungsgefäß zum Zeitpunkt der Zuführung der wäßrigen Probe eine Temperatur unterhalb oder höchstens im Bereich der Siedetemperatur der Probe aufweist und nach deren Zuführung die Temperatur erhöht wird, **dadurch gekennzeichnet, daß** die Probe durch ein und dieselbe Heizeinrichtung in einem ersten Schritt von einer Ausgangstemperatur unterhalb der Siedetemperatur auf eine Verdampfungstemperatur und in einem zweiten Schritt auf eine wesentlich höhere Temperatur erwärmt wird und das Verbrennungsprodukt in einem während der Analyse geschlossenen Kreislauf gehalten wird.

2. Verfahren nach Anspruch 1, **dadurch gekennzeichnet, daß** genau ein als Verbrennungsgefäß ausgebildetes Erhitzungsgefäß eingesetzt wird und die Temperatur im zweiten Schritt auf eine Verbrennungstemperatur erhöht wird.

3. Verfahren nach Anspruch 1, **dadurch gekennzeichnet, daß** der erste und zweite Schritt in einem ersten Erhitzungsgefäß und ein zusätzlicher Verbrennungsschritt in einem zweiten, als Verbrennungsgefäß ausgebildeten Erhitzungsgefäß, das im wesentlichen auf konstanter Verbrennungstemperatur gehalten wird, ausgeführt wird.

4. Verfahren nach Anspruch 2 oder 3, **dadurch gekennzeichnet, daß** die Verdampfungstemperatur im Bereich zwischen 100°C und 300°C, bevorzugt zwischen 100°C und 150°C, und die Verbrennungstemperatur oberhalb 700°C, bevorzugt zwischen 800°C und 1000°C, liegt.

5. Verfahren nach Anspruch 1 oder 2, **dadurch gekennzeichnet, daß** die Erwärmung auf die Verdampfungstemperatur im ersten Schritt in einer Zeitspanne zwischen 10 s und 200 s und die Erwärmung auf die Verbrennungstemperatur im zweiten Schritt in einer Zeitspanne zwischen 5 s und 120 s vorgenommen wird.

6. Verfahren nach einem der vorhergehenden Ansprüche, **dadurch gekennzeichnet, daß** im Anschluß an den ersten Schritt die Temperatur für eine vorbestimmte Zeitspanne, bevorzugt zwischen 10 s und 60 s, im wesentlichen konstant gehalten wird.

7. Verfahren nach einem der vorhergehenden Ansprüche, **dadurch gekennzeichnet, daß** das im zweiten Schritt erhaltene Verbrennungsprodukt einem CO₂-Detektor, insbesondere einem NDIR-Detektor (142; 242; 242), zur Bestimmung des Kohlenstoffgehaltes zugeführt wird.

8. Verfahren nach Anspruch 7, **dadurch gekennzeichnet, daß** den Schritten des Verdampfens und Verbrennens ein Schritt der Separierung anorganischer Kohlenstoffverbindungen aus der Probe, insbesondere durch saure Entgasung, vorgeschaltet ist.

9. Verfahren nach Anspruch 8, **dadurch gekennzeichnet, daß** in Verbindung mit dem Schritt der Separierung der anorganischen Kohlenstoffverbindungen eine Bestimmung des Gehalts an anorganischem Kohlenstoff, insbesondere durch Zuführung des Entgasungsprodukts zu dem CO₂-Detektor vor dem Schritt des Verbrennens, durchgeführt wird.

10. Verfahren nach einem der vorhergehenden Ansprüche, **dadurch gekennzeichnet, daß** das Verbrennungsprodukt einem Stickstoffdetektor, insbesondere einem Chemolumineszenzdetektor, zur Bestimmung des Stickstoffgehaltes zugeführt wird.

11. Verfahren nach einem der vorhergehenden Ansprüche, **dadurch gekennzeichnet, daß** dem Schritt des Verdampfens ein Schritt der Separierung von Halogenid-, insbesondere Chlorid-Ionen nachgeschaltet ist.

12. Verfahren nach Anspruch 11, **dadurch gekennzeichnet, daß** in Verbindung mit dem Schritt der Separierung eine Gehaltsbestimmung der Halogenidionen, insbesondere mittels eines coulometrischen Verfahrens, durchgeführt wird.

13. Verfahren nach einem der vorhergehenden Ansprüche, **dadurch gekennzeichnet, daß** das im Schritt des Verdampfens erhaltene Gas, das im wesentlichen aus Wasserdampf besteht, in einer Kühlfalle kondensiert wird.

14. Verfahren nach Anspruch 13 und Anspruch 11 oder 12, **dadurch gekennzeichnet, daß** der Schritt der Separierung von Halogenidionen in einem geeigneten Agens, insbesondere Ag oder AgNO₃, in der Kühlfalle ausgeführt wird.

15. Vorrichtung zur Durchführung des Verfahrens nach einem der vorhergehenden Ansprüche, **dadurch gekennzeichnet, daß** die Heizeinrichtung des oder des ersten Erhitzungsgefäßes durch einen optischen Strahlungsofen (154; 154'; 254; 254) gebildet ist, der mit einer schnell ansprechenden Temperatur-Zeit-Steuerung (146; 256; 356) versehen ist.

16. Vorrichtung nach Anspruch 15, **dadurch gekennzeichnet, daß** der Strahlungsofen (154') eine Mehrzahl von auf einer Zylindermantelfläche um das Verbrennungsgefäß (154a') herum angeordneten Halogenheizstäben (154b') und einen die Halogenheizstäbe umgebenden elliptischen Reflektor (154d') aufweist.

17. Vorrichtung nach Anspruch 15, **dadurch gekennzeichnet, daß** der Strahlungsofen einen zylindrischen Reflektor mit elliptischem Querschnitt (154d) aufweist, in dessen einer Brennlinie das Verbrennungsgefäß (154a) und in dessen anderer Brennlinie ein Halogenheizstab (154b) angeordnet ist.

18. Vorrichtung nach einem der Ansprüche 15 bis 17, **gekennzeichnet durch** die Ausbildung als gegenüber der Atmosphäre abschließbares verzweigtes System (100; 200; 300), in dem mindestens eine das Verbrennungsgefäß (154a; 254a; 354a) mit dem Detektor (142; 242; 342) verbindende Ringleitung (140b, 140, 128b, 114; 240b, 240, 228b, 214; 340b, 340, 328b, 314) vorgesehen ist.

19. Vorrichtung nach Anspruch 18, **gekennzeichnet durch** eine ausgangsseitig mit dem Detektor (142; 242; 342) verbundene Entgasungseinrichtung (122; 222; 322) zur sauren Entgasung der Probe.

20. Vorrichtung nach Anspruch 19, **gekennzeichnet durch** einen in eine Leitung zwischen der Entgasungseinrichtung und dem Detektor einschleifbaren CO₂-Fänger (132; 332).

## Claims

1. A method for determining a constituent in water, in particular the content of organic carbon and/or nitrogen, in which an aqueous sample is evaporated and combusted in at least one heating vessel (154a; 154a'; 254; 354) provided with a heater (154; 154'; 254; 354), and the combustion product is delivered in a transporting gas stream to a detector (142; 242; 342) for determining the concentration of a gaseous compound of the constituent, wherein the heating vessel, or a first heating vessel, at the instant of delivery of the aqueous sample has a temperature below or at most at about the boiling temperature of the sample, and after delivery of the sample the temperature is increased, **characterized in that** the sample is heated by one and the same heater in a first step from an outset temperature below the boiling temperature to an evaporation temperature, and in a second step to a substantially higher temperature, and the combustion product is kept in closed circulation during the analysis.

2. The method of claim 1, **characterized in that** precisely one heating vessel embodied as a combustion vessel is used, and the temperature in the second step is increased to a combustion temperature.

3. The method of claim 1, **characterized in that** the first and second steps are performed in a first heating vessel, and an additional combustion step is performed in a second heating vessel, embodied as a combustion vessel, which is kept substantially at constant combustion temperature.

4. The method of claim 2 or 3, **characterized in that** the evaporation temperature is in the range between 100°C and 300°C, preferably between 100°C and 150°C, and the combustion temperature is above 700°C, preferably between 800°C and 1000°C.

5. The method of claim 1 or 2, **characterized in that** the heating to the evaporation temperature is effected in the first step in a period of time of between 10 s and 200 s, and the heating to the combustion temperature is effected in the second step within a period of time of between 5 s and 120 s.

6. The method of one of the preceding claims, **characterized in that** following the first step, the temperature is kept substantially constant for a predetermined time period, preferably between 10 s and 60 s.

7. The method of one of the preceding claims, **characterized in that** the combustion product obtained in the second step is delivered to a CO₂ detector, in particular an NDIR detector (142; 242; 242) for determining the carbon content.

8. The method of claim 7, **characterized in that** the steps of evaporation and combustion are preceded by a step of separating inorganic carbon compounds from the sample, in particular by acidic degassing.

9. The method of claim 8, **characterized in that** in combination with the step of separating the inorganic carbon compounds, a determination of the content of inorganic carbon is performed, in particular by delivering the degassing product to the CO₂ detector prior to the step of the combustion.

10. The method of one of the preceding claims, **characterized in that** the combustion product is delivered to a nitrogen detector, in particular a chemoluminescence detector, for determining the nitrogen content.

11. The method of one of the preceding claims, **characterized in that** the step of evaporation is followed by a step of separating halide ions, in particular chloride ions.

12. The method of claim 11, **characterized in that** in combination with the separation step, a quantitative determination of the halide ions is performed, in particular by means of a coulometric method.

13. The method of one of the preceding claims, **characterized in that** the gas obtained in the step of the evaporation, which substantially comprises water vapor, is condensed in a cold trap.

14. The method of claim 13 and claim 11, **characterized in that** the step of the separation of halide ions is done in a suitable agent, in particular Ag or AgNO₃ in the cold trap.

15. An apparatus for performing the method of one of the preceding claims, **characterized in that** the heater of the heating vessel or of the first heating vessel is formed by an optical radiant oven (154; 154'; 254; 254), which is provided with a fast-response temperature-time controller (146; 256; 356).

16. The apparatus according to claim 15, **characterized in that** the radiant oven (154') has a plurality of halogen heating bars (154b'), disposed on a cylinder jacket face around the combustion vessel (154a'), and an elliptical reflector (154d') surrounding the halogen heating bars.

17. The apparatus according to claim 15, **characterized in that** the radiant oven has a cylindrical reflector of elliptical cross section (154d), in one focal line of which the combustion vessel (154a) is disposed, and in the other focal line of which a halogen heating bar (154b) is disposed.

18. The apparatus according to one of claims 15 to 17, **characterized by** the implementation as a branching system (100; 200; 300) which can be closed off from the atmosphere and in which at least one ring line (140b, 140, 128b, 114; 240b, 240, 228b, 214; 340b, 340, 328b, 314) connecting the combustion vessel (154a; 254a; 354a) with the detector (142; 242; 342) is provided.

19. The apparatus according to claim 18, **characterized by** a degassing device (122; 222; 322), communicating on the outlet side with the detector (142; 242; 342), for an acidic degassing of the sample.

20. The apparatus according to claim 19, **characterized by a** CO₂ collector (132; 332) that can be looped into a line between the degassing device and the detector.

## Revendications

1. Procédé pour la détermination d'une substance contenue dans de l'eau, en particulier du contenu en carbone et/ou azote organique/s, dans lequel un échantillon aqueux est évaporé et brûlé dans un récipient de chauffage (154a ; 154a' ; 254 ; 354) muni d'au moins un moyen de chauffage (154 ; 154' ; 254 ; 354), et le produit de la combustion est amené dans un flux de gaz de transport à un détecteur (142 ; 242 ; 342) pour la détermination de la concentration d'un composé gazeux de la substance contenue, l'un ou un premier récipient de chauffage présentant au moment de l'alimentation de l'échantillon aqueux une température au-dessous de ou au maximum dans le rayon de la température d'ébullition de l'échantillon et qui est augmentée après alimentation de l'échantillon, **caractérisé en ce que** l'échantillon est chauffé par le même moyen de chauffage dans une première étape partant d'une température initiale au-dessous de la température d'ébullition à une température d'évaporation, et est chauffé dans une deuxième étape à une température considérablement plus élevée, et que le produit de combustion est retenu dans un circuit qui est fermé pendant l'analyse.

2. Procédé selon la revendication 1, **caractérisé en ce qu**'exactement un récipient de chauffage configuré comme récipient de combustion est utilisé, et que la température est augmentée à une température de combustion dans la deuxième étape.

3. Procédé selon la revendication 1, **caractérisé en ce que** la première et la deuxième étape sont réalisées dans un premier récipient de chauffage, et une étape additionnelle de combustion est réalisée dans un deuxième récipient de chauffage configuré comme récipient de combustion et qui est essentiellement maintenu à une température de combustion constante.

4. Procédé selon la revendication 2 ou 3, **caractérisé en ce que** la température d'évaporation est comprise dans le rayon entre 100°C et 300°C, de préférence entre 100°C et 150°C, et que la température de combustion est au-dessus de 700°C, de préférence entre 800°C et 1000°C.

5. Procédé selon les revendications 1 ou 2, **caractérisé en ce que** l'échauffement à la température d'évaporation dans la première étape est effectué dans une période de temps comprise entre 10 s et 200 s, et que l'échauffement à la température de combustion dans la deuxième étape est effectué dans une période de temps comprise entre 5 s et 120 s.

6. Procédé selon l'une quelconque des revendications précédentes, **caractérisé en ce qu**'à la suite de la première étape, la température est maintenue essentiellement constante pendant une période de temps prédéterminée, de préférence comprise entre 10 s et 60 s.

7. Procédé selon l'une quelconque des revendications précédentes, **caractérisé en ce que** le produit de combustion obtenu dans la deuxième étape est amené à un détecteur de CO₂, en particulier un détecteur IRND (142 ; 242 ; 242), pour la détermination du contenu en carbone.

8. Procédé selon la revendication 7, **caractérisé en ce que** les étapes d'évaporation et de combustion sont précédées d'une étape de séparation des composés inorganiques du carbone de l'échantillon, en particulier par dégazage acide.

9. Procédé selon la revendication 8, **caractérisé en ce qu**'en combinaison avec l'étape de séparation des composés inorganiques du carbone, une détermination du contenu en carbone inorganique est effectuée, en particulier par l'alimentation du détecteur de CO₂ avec le produit de dégazage avant l'étape de combustion.

10. Procédé selon l'une quelconque des revendications précédentes, **caractérisé en ce que** le produit de combustion est amené à un détecteur d'azote, en particulier un détecteur de chimioluminescense pour la détermination du contenu en azote.

11. Procédé selon l'une quelconque des revendications précédentes, **caractérisé en ce que** l'étape d'évaporation est suivie d'une étape de séparation des ions de haloïde, en particulier des ions de chlorure.

12. Procédé selon la revendication 11, **caractérisé en ce qu**'en combinaison avec l'étape de séparation, une détermination du contenu en ions de haloïde est effectuée, en particulier par un procédé coulométrique.

13. Procédé selon l'une quelconque des revendications précédentes, **caractérisé en ce que** le gaz obtenu dans l'étape d'évaporation consistant essentiellement en vapeur d'eau, est condensé dans un piège cryogénique.

14. Procédé selon la revendication 13 et les revendication 11 ou 12, **caractérisé en ce que** l'étape de séparation des ions de haloïde est effectuée dans le piège cryogénique dans un agent appropriée, en particulier Ag ou AgNO₃.

15. Dispositif pour réaliser le procédé selon l'une quelconque des revendications précédentes, **caractérisé en ce que** le moyen de chauffage du ou du premier récipient de chauffage est formé par un radiateur optique (154 ; 154' ; 254 ; 254) équipé d'une commande de température et de temps (146 ; 256 ; 356) à réponse rapide.

16. Dispositif selon la revendication 15, **caractérisé en ce que** le radiateur (154') comporte une pluralité de bâtons de chauffage halogène (154b') disposés autour du récipient de combustion (154a') sur une enveloppe du cylindre, et un réflecteur elliptique (154d') entourant les bâtons de chauffage halogène.

17. Dispositif selon la revendication 15, **caractérisé en ce que** le radiateur comporte un réflecteur cylindrique ayant une section transversale elliptique (154d), dans l'une de ses lignes focales un récipient de combustion (154a) est disposé, et dans l'autre de ses lignes focales un bâton de chauffage halogène (154b).

18. Dispositif selon l'une quelconque des revendications 15 à 17, **caractérisé par** la configuration en tant que système branché (100 ; 200 ; 300) qui peut être isolé de l'atmosphère et dans lequel est prévue au moins une conduite circulaire (140b, 140, 128b, 114 ; 240b, 240, 228b, 214 ; 340b, 340, 328b, 314) reliant le récipient de combustion (154a ; 254a ; 354a) au détecteur (142 ; 242 ; 342).

19. Dispositif selon la revendication 18, **caractérisé par** un moyen de dégazage (122 ; 222 ; 322) relié côté sortie au détecteur (142 ; 242 ; 342) pour le dégazage acide de l'échantillon.

20. Dispositif selon la revendication 19, **caractérisé par** un dispositif de captage du CO₂ (132 ; 332) qui peut être bouclé dans un conduit entre le moyen de dégazage et le détecteur.
